(19) Europäisches Patentamt
European Patent Office
Office européen des brevets

(11) **EP 1 777 522 A1**

(12) **EUROPEAN PATENT APPLICATION**

(43) Date of publication:
**25.04.2007 Bulletin 2007/17**

(51) Int Cl.:
***G01N 33/569*** *(2006.01)*    ***B01L 3/00*** *(2006.01)*

(21) Application number: **06122690.8**

(22) Date of filing: **20.10.2006**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(30) Priority: **20.10.2005 JP 2005306419
20.10.2005 JP 2005306420**

(71) Applicant: **Pentax Corporation
Tokyo 174-8639 (JP)**

(72) Inventors:
• **Yagasaki, Kazumi
Itabashi-ku,
Tokyo 174-8639 (JP)**
• **Miyazaki, Isao
Itabashi-ku,
Tokyo 174-8639 (JP)**

(74) Representative: **Schaumburg, Thoenes, Thurn,
Landskron
Patentanwälte
Postfach 86 07 48
81634 München (DE)**

(54) **Plate, kit and method for the determination of a virus infection**

(57)    Disclosed herein are a plate capable of determining the presence or absence of an anti-virus antibody with higher accuracy, a determination kit equipped with the plate, and a determination method using the determination kit for immunoassay. The determination method is as follows. First, a liquid specimen from a living body is fed into each of the predetermined wells of the plate.

Each well carries one of the two or more kinds of antigens derived from a specific virus on its inner surface. Then, a reagent containing colored particles carrying an anti-IgG antibody is fed into each of the wells containing the liquid specimen. When an anti-virus antibody bound to the antigen is detected in a predetermined number or more of the wells, a living body is determined to be infected with the virus.

FIG. 1

EP 1 777 522 A1

**Description**

## BACKGROUND OF THE INVENTION

### Field of the Invention

The present invention relates to a plate, a determination kit, and a determination method for immunoassay, and more specially relate to a plate capable of determining the presence or absence of an anti-virus antibody with higher accuracy, a determination kit equipped with the plate, and a determination method using the determination kit for immunoassay.

Heretofore, various diagnostic kits utilizing immunoassay based on an antigen-antibody reaction, such as enzyme-linked immunosorbent assay, radioimmunoassay, and particle agglutination (e.g., see USP 5,540,995) have been developed.

One of the common requirements for these diagnostic kits is detection accuracy (reliability). Particularly, in the case of diagnosis of virus infection associated with human rights, such as HIV infection, the detection accuracy of diagnostic kits for detecting such virus infection needs to be very high.

However, in reality, about two or three persons per 1,000 persons usually test pseudo-positive for the anti-HIV antibody in spite of the fact that they are not infected with HIV (i.e., they are negative for HIV infection). The accuracy of such diagnostic kits needs to be substantially 100 %, but under the present circumstances, it is difficult to achieve such high accuracy.

## SUMMARY OF THE INVENTION

It is therefore an object of the present invention to provide a plate capable of determining the presence or absence of the anti-virus antibody with higher accuracy, a determination kit equipped with the plate, and a determination method using the determination kit which facilitates the operation of determining the presence or absence of the anti-virus antibody.

In order to achieve the above object, the present invention is directed to a plate to be used for determining whether or not a living body is infected with a specific virus, comprising:

a plurality of wells into which a liquid specimen containing a sample obtained from the living body is to be fed, each of the wells having an inner surface; and
two or more kinds of antigens derived from the virus, wherein each of the wells carries one of the two or more kinds of antigens on the inner surface thereof.

Such a plate is capable of determining the presence or absence of the anti-virus antibody with higher accuracy.

In the plate of the present invention, it is preferred that the plurality of wells include a plurality of wells arranged in line into which the same liquid specimen is to be fed.

This facilitates the operation of feeding (dispensing) a liquid specimen into the wells. Further, the state of the inside of each well can be easily observed, thereby facilitating the determination of the presence or absence of the anti-virus antibody.

Further, in the plate of the present invention, it is also preferred that the virus is HIV (Human Immunodeficiency Virus).

In the case of determination of virus infection or uninfection associated with human rights, such as HIV infection or uninfection, the possibility of error in determination is preferably minimized. Therefore, the plate of the present invention capable of determining the presence or absence of the anti-virus antibody with higher accuracy is particularly suitable for use in determination of HIV infection or uninfection.

Another aspect of the present invention is directed to a determination kit to be used for determining whether or not a living body is infected with a specific virus, comprising:

the plate described above; and
a reagent capable of detecting an antibody bound to the antigen.

Such a determination Kit is capable of determining the presence or absence of the anti-virus antibody with higher accuracy.

In the determination kit of the present invention, it is preferred that the reagent contains particles carrying an anti-IgG antibody.

Such a method, that is, particle agglutination is advantageous because it is a simple and sensitive method which requires fewer steps and is capable of determining the presence or absence of the anti-virus antibody in a short period of time without using special devices or equipment.

Further, in the determination kit of the present invention, it is also preferred that the particles are colored.

This is advantageous in that the determination of the presence or absence of the anti-virus antibody can be made under visible light irradiation.

Furthermore, in the determination kit of the present invention, it is also preferred that the bottom of each well in the plate is made convergent.

This allows the sedimented particles to be rolled toward the central portion of the well and then to be sedimented and gathered together. As a result, in a case where the particles contained in the reagent are colored, these colored particles can be visually recognized by their hue of a deep color at the central portion of the well (i.e., the central portion of the well is highly colored) thereby facilitating the recognition of sedimentation and agglutination (i.e., motion and state) of the colored particles in the well.

Yet another aspect of the present invention is directed to a determination method for determining whether or not a living body is infected with a specific virus using the determination kit described above, the method comprising:

feeding the liquid specimen into each of the predetermined wells in the plate;
feeding the reagent into each of the wells containing the liquid specimen; and
determining that the living body is infected with the virus when an antibody bound to the antigen is detected in a predetermined number or more of the wells.

Such a determination method is capable of determining the presence or absence of the anti-virus antibody with higher accuracy.

In the determination method of the present invention, it is preferred that when the number of kinds of the antigens is defined as "A" and the predetermined number of the wells at which the living body is to be determined as being infected with the virus is defined as "B", B/A is 0.2 or higher.

This improves the correctness of the result of determination (i.e., accuracy of determination) of the presence or absence of the anti-virus antibody in a living body.

## BRIEF DESCRIPTION OF THE DRAWINGS

Fig. 1 is a perspective view which schematically shows a plate of a determination kit of the present invention;
Fig. 2 is a plan view of the plate shown in Fig. 1;
Figs. 3(a) and 3(b) are illustrations for explaining a determination method of the present invention;
Fig. 4 is an illustration which shows results determined by the determination method of the present invention;
Fig. 5 is an illustration for explaining the determination method of the present invention;
Fig. 6 is an illustration for explaining the determination method of the present invention; and
Fig. 7 is a photograph which shows results determined in the Example and the Comparative Example.

## DETAILED DESCRIPTION OF THE INVENTION

Hereinbelow, a plate, a determination kit, and a determination method according to the present invention will be described in detail with reference to a preferred embodiment shown in the accompanying drawings.

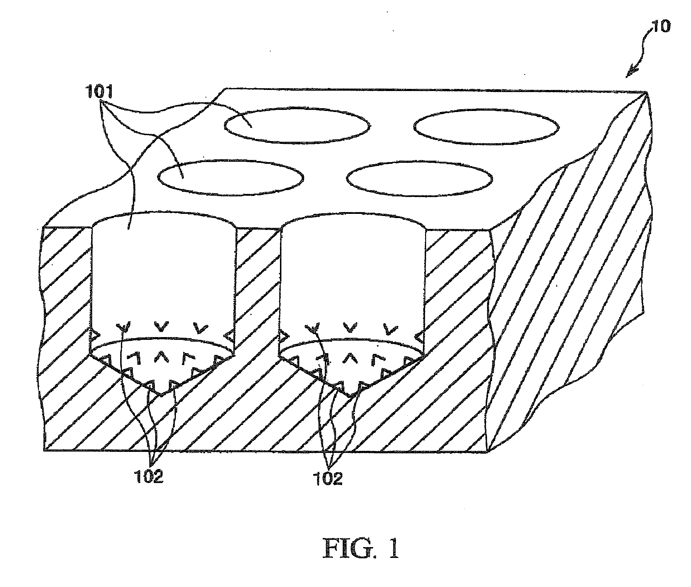

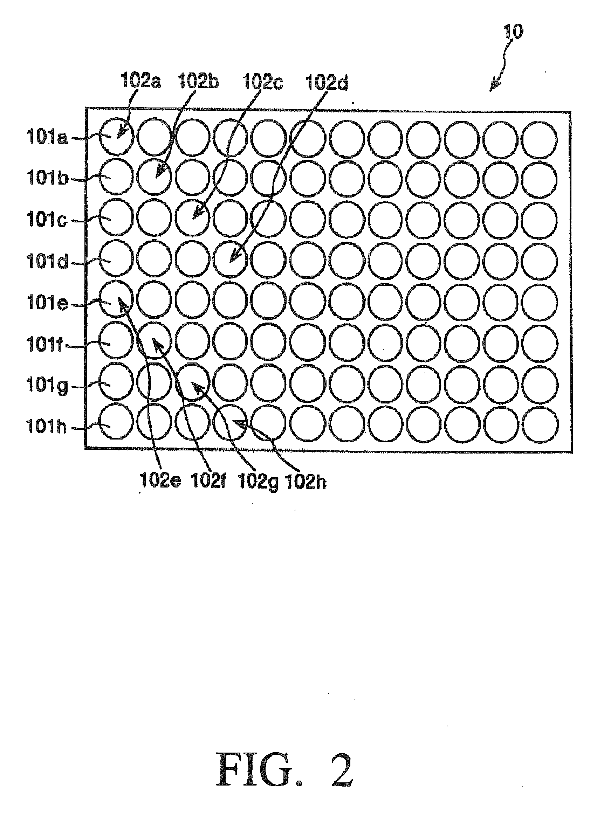

Fig. 1 is a perspective view which schematically shows a plate of a determination kit of the present invention, and Figs. 2 to 4 are illustrations for explaining a determination method of the present invention.

First, the determination kit of the present invention will be described.

The determination kit of the present invention is used to determine whether or not a living body is infected with a specific virus by detecting the presence or absence of an antibody against the specific virus (i.e., an anti-virus antibody) in a sample obtained from the living body.

Examples of a method for detecting such an antibody include particle agglutination (PA), enzyme-linked immunosorbent assay (ELISA), and radioimmunoassay (RIA). Among them, particle agglutination is preferably employed because it is a simple and sensitive method which requires fewer steps and is capable of determining the presence or absence of the anti-virus antibody in a short period of time without using special devices or equipment.

Hereinbelow, a case where the detection of an antibody is carried out using particle agglutination will be described by way of example.

The detection of an antibody (i.e., a determination as to whether or not a living body is infected with a specific virus) using particle agglutination is carried out by, for example, the following method.

First, a liquid specimen containing a sample obtained from a living body is fed into a well whose inner surface carries an antigen derived from a target virus. Then, a reagent containing colored particles carrying an anti-IgG antibody is fed into the well to determine whether an anti-virus antibody is present (positive) or absent (negative) in the liquid specimen (i.e., in the sample).

The determination kit of the present invention is used for such determination of the presence or absence of the anti-virus antibody. The determination kit includes a plate 10 (i.e., a plate according to the present invention) equipped with a plurality of wells 101 each carrying an antigen 102 derived from a virus, and a reagent (i.e., a reagent for determination) containing colored particles 1 carrying an anti-IgG antibody 104.

As shown in Fig. 1, the plate 10 is formed from a flat plate-shaped member, and has a plurality of wells 101 arranged in a matrix in one of the surfaces thereof.

Examples of a material for forming the plate 10 include resin materials such as polystyrene, polycarbonate, and polypropylene. These resin materials can be used singly or in combination of two or more of them.

The well 101 provides space for holding (storing) the liquid specimen and the reagent, and carries an antigen 102 on the inner surface thereof.

As shown in Fig. 1, the well 101 has a shape such that the bottom thereof is made convergent (i.e., in this embodiment, the depth of the well 101 becomes deeper toward the central portion of the well 101). This allows the sedimentated colored particles 1 to be rolled toward the central portion of the well 101 and then to be gathered together and agglutinated. As a result, the colored particles 1 can be visually recognized by their hue of a deep color at the central portion of the well 101 (i.e., the central portion of the well 101 is highly colored), thereby facilitating the recognition of sedimentation and agglutination (i.e., motion and state) of the colored particles 1 in the well 101.

As described above, the well 101 preferably has a shape such that the bottom thereof is made convergent. However, unlike the well 101 shown in Fig.1, the deepest point of the well 101 may be off the central portion of the well 101.

The capacity of the well 101 is not particularly limited, but is preferably in the range of about 0.03 to 0.5 mL, and more preferably in the range of about 0.05 to 0.3 mL. By setting the capacity of the well 101 to a value within the above range, it is possible to hold a liquid (i.e., the liquid specimen and the reagent) in an amount necessary and sufficient to make determination of the presence or absence of the anti-virus antibody without increasing the size of the plate 10.

The antigen 102 is fixed (carried) on the inner surface of the well 101 by, for example, hydrophobic bonding.

In the present invention, two or more kinds (in this embodiment, 8 kinds) of virus-derived antigens 102 are used, and each well 101 carries one of the antigens 102 on the inner surface thereof.

For example, in the plate 10 shown in Fig. 2, a first antigen 102a is fixed on the inner surface of a first well 101a, and a second antigen 102b is fixed on the inner surface of a second well 101b. Similarly, third to eighth antigens 102c to 102h are fixed on the inner surfaces of third to eighth wells 101c to 101h, respectively.

By doing so, it is possible to accurately determine whether or not a living body is infected with a specific virus, which will be described later in more detail with reference to the determination method of the present invention.

Further, in the plate 10 shown in Fig. 2, the wells 101a to 101h into which the same liquid specimen is to be fed are arranged in line (that is, in an array of wells), thereby facilitating the operation of feeding (dispensing) a liquid specimen into each of the wells 101a to 101h. In addition, the state of the colored particles in each of the wells 101a to 101h can be visually recognized with ease, thereby facilitating the determination of the presence or absence of the anti-virus antibody.

The antigens to be used are selected according to a target virus and are not particularly limited. For example, in a case where a target virus is HIV (Human Immunodeficiency Virus), examples of an antigen to be used include HIV-1 p18, HIV-1 p24, HIV-1 p55, HIV-1 p31, HIV-1 p51, HIV-1 p66, HIV-1 gp41, HIV-1 gpl20, HIV-1 gp160, HIV-2 p16, HIV-2 p26, HIV-2 p56, HIST-2 p34, HIV-2 p53, HIV-2 p68, HIV-2 gp36, HIV-2 gp105, and HIV-2 gp140. Among them, two or more kinds of antigens can be selected.

The amount of the antigen 102 to be fixed on the inner surface of the well 101 is preferably 0.01 $\mu$g or more, and more preferably in the range of about 0.05 to 1 $\mu$g. By setting the amount of the antigen 102 to a value within the above range, it is possible to allow a sufficient amount of an anti-virus antibody 103 to bind to the antigen 102 when the anti-virus antibody 103 is present in the sample (i.e., in the liquid specimen), thereby enabling the detection of the anti-virus antibody 103 to be carried out more accurately. It is to be noted that even if the amount of the antigen 102 is increased to exceed the above upper limit value, it cannot be expected that the accuracy of detection is further improved.

In this embodiment, the reagent is a suspension containing colored particles 1 carrying an anti-IgG antibody 104.

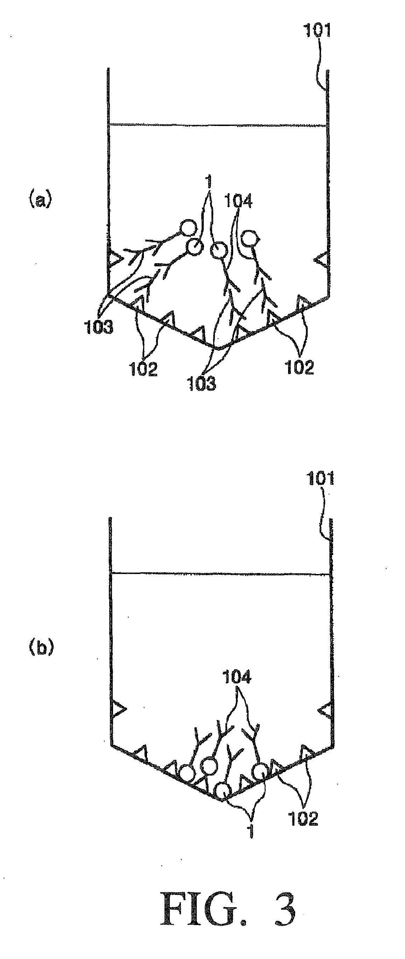

When such a reagent is used, as shown in Fig. 3(a), the anti-IgG antibody 104 (i.e., a labeled secondary antibody) carried on the colored particles 1 is bound to an anti-virus antibody 103 (i.e., a primary antibody) in a case where the anti-virus antibody 103 has been bound to the antigen 102. As a result, the colored particles 1 are adsorbed to the inner surface of the well 101 through the anti-IgG antibodies 104, the anti-virus antibodies 103 and the antigens 102 with the colored particles 1 being dispersed in the liquid in the well 101.

On the other hand, as shown in Fig. 3(b), in a case where an anti-virus antibody 103 is not bound to the antigen 102, the colored particles 1 are sedimentated and agglutinated in the well 101.

Namely, a state where the colored particles 1 contained in the reagent fed into the well 101 are adsorbed to the inner surface of the well 101 through the anti-IgG antibodies 104, the anti-virus antibodies 103 and the antigens 102 with the colored particles 1 being dispersed in the liquid in the well 101. This state shows that an anti-virus antibody 103 bound to the antigen 102 fixed on the inner surface of the well 101 is present. On the other hand, a state where the colored particles 1 contained in the reagent fed into the well 101 are sedimentated in the well 101 shows that an anti-virus antibody 103 against the antigen 102 fixed on the inner surface of the well 101 is not present.

It is to be noted that in this embodiment, colored particles 1 are used as particles contained in the reagent. However, in the present invention, other particles such as particles carrying a fluorescent material may be alternatively used. The merit of using colored particles 1 as particles contained in the reagent is that the determination of the presence or absence of the anti-virus antibody can be made under visible light irradiation.

Such particles (colored particles 1) can be formed using a resin material, a ceramic material, or the like. Alternatively, composite particles composed of a resin material and a ceramic material (e.g., resin particles coated with a ceramic material) may be used.

Examples of the resin material include thermoplastic resins such as polyamide (e.g., nylon 6, nylon 6.6, nylon 6.10, nylon 12), polyethylene, polypropylene, polystyrene, polyimide, methacrylic resins, acrylic resins, and thermoplastic polyurethanes, and thermosetting resins such as epoxy resins, phenol resins, melamine resins, urea resins, unsaturated polyesters, alkyd resins, thermosetting polyurethanes, and ebonite.

Examples of the ceramic material include calcium phosphate-based compounds such as $Ca_{10}(PO_4)_6(OH)_2$, $ca_{10}(PO_4)_6F_2$, $Ca_{10}(PO_4)_6Cl_2$, $Ca_3(PO_4)_2$, $Ca_2P_2O_7$, $Ca(Pl_3)_2$, and $CaHPO_4$, alumina, and silica.

The above-described composite particles can be produced by, for example, colliding ceramic porous particles with the surface of resin particles by the use of a commercially-available hybridization machine or mechanofusion machine.

The specific gravity of the particles is preferably larger than that of a liquid present in the well 101 in Step <5> which will be described later. More specifically, the specific gravity of the particles is preferably in the range of about 1.05 to 1.3 g/cm$^3$. By setting the specific gravity of the particles to a value within the above range, it is possible to speedily and reliably precipitate and agglutinate the particles in the well 101 when necessary (i.e., when a liquid specimen should be determined to be negative).

The average particle diameter of the particles is preferably 30 μm or less, and more preferably in the range of about 1 to 20 μm.

In a case where colored particles 1 are used as particles contained in the reagent, they can be prepared by staining particles with a dye or pigment. Examples of the dye include direct dyes, acid dyes, basic dyes, mordant dyes, acid mordant dyes, sulfur dyes, sulfur vat dyes, vat dyes, soluble vat dyes, azoic dyes, disperse dyes, reactive dyes, oxidation dyes, dyes for synthetic fibers, and fluorescent whitening dyes.

On the surface of the colored particles 1 (particles), an anti-IgG antibody 104 is immobilized (carried) directly or with the aid of an immobilizing agent.

Examples of the immobilizing agent include glutaraldehyde, formaldehyde, coupling agents, and osmium tetrachloride. Examples of the coupling agents include 3-glycidoxypropyl trimethoxysilane, 3-thiopropyl trimethoxysilane, 2-(3-trimethoxysilylpropyldithio)-5-nitropyridtne, 3-aminopropyl triethoxysilane, and 3-chloropropyl dimethoxymethylsilane.

In a case where at least the surface of each of the colored particles (colored beads) 1 and its vicinity are made of a calcium phosphate-based compound, part of the surface of each of the colored particles 1 is preferably blocked (coated) with a blocking agent after the anti-IgG antibody 104 is immobilized on the surface of each of the colored particles 1. As such a blocking agent, one having no effect on an antigen-antibody reaction, such as casein or albumin, is preferably used.

The amount of the colored particles 1 to be contained in the reagent is not particularly limited, but is preferably in the range of about 0.05 to 0.2 wt%, and more preferably in the range of about 0.08 to 0.12 wt%. By setting the amount of the colored particles 1 to be contained in the reagent to a value within the above range, it is possible to visually recognize the motion and state of the colored particles 1 in the well 101 with reliability. If the amount of the colored particles 1 contained in the reagent is too much, there is a case that some inconveniences, such as clogging of a pipet tip, occur when the reagent is fed (dispensed) into the wells 101.

Examples of a dispersion medium to be used for preparing the reagent include various buffers such as triethanolamine hydrochloride-sodium hydroxide buffer, veronal (sodium 5,5-diethyl barbiturate)-hydrochloride buffer, Tris-hydrochloride buffer, glycylglycine-sodium hydroxide buffer, 2-amino-2-methyl-1,3-propanediol-hydrochloride buffer, diethanolamine-hydrochloride buffer, boric acid buffer, sodium borate-hydrochloride buffer, glycine-sodium hydroxide buffer, sodium carbonate-sodium bicarbonate buffer, sodium borate-sodium hydroxide buffer, sodium bicarbonate-sodium hydroxide buffer, potassium dihydrogen phosphate-disodium hydrogen phosphate buffer, disodium phosphate-sodium hydroxide buffer, potassium chloride-sodium hydroxide buffer, Britton-Robinson buffer, and GTA buffer, and those obtained by adding normal saline to these buffers.

Next, a method for using such a determination kit described above, that is, a method for determining whether or not a living body is infected with a specific virus (i.e., a determination method of the present invention) will be described.

<1> First, a liquid specimen to be subjected to a determination of the presence or absence of the anti-virus antibody is prepared. As such a liquid specimen, a sample obtained from a living body can be directly used. If necessary, the volume of the sample may be adjusted by, for example, dilution before use.

Examples of a liquid to be used for adjusting the volume of the sample include the buffers mentioned above with reference to the preparation of a reagent.

Examples of the sample include liquids such as blood serum (blood), semen, vaginal secretion, breast milk, saliva, urine, cerebrospinal fluid, and lymph, and solids (solid matter) such as cells (tissues). It is to be noted that in a case where a solid sample such as cells is used, a liquid specimen can be prepared by crushing the sample and suspending the crushed sample in the buffer.

In a case where blood serum is used as a sample, a liquid specimen is preferably prepared by diluting blood serum about 2- to 1000-fold (especially, about 5- to 100-fold).

<2> Next, a plate 10 having wells 101 (101a to 101h) is prepared. It is to be noted that each of the wells 101 (101a to 101h) carries one of two or more kinds of antigens 102 (102a to 102h) on the inner surface thereof. Then, the wells 101 are cleaned with a cleaning solution.

Examples of such a cleaning solution include the buffers mentioned above with reference to the preparation of a reagent.

<3> Next, the same liquid specimen is fed (supplied) into each of the wells 101 (101a to 101h) arranged in line.

In a case where the liquid specimen contains an anti-virus antibody 103, as shown in Fig. 3(a), the anti-virus antibody 103 contained in the liquid specimen fed into the well 101 is bound to (captured by) the antigen 102 fixed on the inner surface of the well 101.

It is to be noted that the amount of the liquid specimen to be fed into each well 101 is not particularly limited, but is preferably in the range of about 0.02 to 0.25 mL, and more preferably in the range of about 0.04 to 0.06 mL.

<4> Next, the liquid specimen is removed from each of the wells 101, and then the wells 101 are cleaned with the same cleaning solution as that mentioned above.

By doing so, it is possible to remove an interfering substance or the like which interferes with the detection of an anti-virus antibody 103, thereby enabling the detection of an anti-virus antibody 103 to be more accurately carried out.

<5> Next, a reagent is fed into each of the wells 101. As shown in Fig. 3(a), in a case where an anti-virus antibody 103 has been bound to the antigen 102, an anti-IgG antibody 104 fixed on colored particles 1 contained in the reagent fed into the well 101 is bound to the anti-virus antibody 103. As a result, the colored particles 1 are adsorbed to the inner surface of the well 101 through the anti-IgG antibodies 104, the anti-virus antibodies 103 and the antigens 102 with the colored particles 1 being dispersed in the liquid in the well 101 (i.e., in the reagent) so that the liquid contained in the well 101 appears to be lightly colored. This state shows that an anti-virus antibody 103 bound to the antigen 102 fixed on the inner surface of the well 101 is present.

On the other hand, as shown in Fig. 3(b), in a case where no anti-virus antibody 103 is bound to the antigen 102, the colored particles 1 carrying the anti-IgG antibody 104 are sedimentated and agglutinated. As a result, an area highly colored (in this embodiment, a spot) is generated at the bottom of the well 101. This state shows that a specific anti-virus antibody 103 bound to the antigen 102 fixed on the inner surface of the well 101 is not present.

Meanwhile, a living body has a plurality kinds of antibodies against various antigens. Therefore, there is a case where some of the antibodies are accidentally bound to (cross-react with) the first to eighth antigens 102a to 102h in spite of the fact that they are not antibodies produced against the first to eighth antigens 102a to 102h. Also in a case where such antibodies are bound to the first to eighth antigens 102a to 102h, the colored particles 1 are adsorbed to the inner surface of the well in the wells 101a to 101h through the anti-IgG antibodies 104, the antibodies and the antigens 102 with the colored particles 1 being dispersed in the liquid in the wells 101a to 101h. This causes a mistaken determination that anti-virus antibodies 103 against the first to eighth antigens 102a to 102h are present.

However, there are not so many antibodies which are generally present in a living body and will cross-react with special antigens, that is, with the first to eighth antigens 102a to 102h.

Therefore, in a case or when the number of wells from which the antibody bound to the antigen 102 (102a to 102h) has been detected is equal to or greater than a predetermined number (cut line), the liquid specimen shall be determined to be positive, that is, it shall be determined that the living body is infected with a specific virus.

By setting such a cut line, it is possible to reliably prevent a negative liquid specimen from being misjudged to be positive, thereby enabling the determination of the presence or absence of the anti-virus antibody in a living body to be made with accuracy.

Here, when the number of kinds of the antigens 102 to be used is defined as "A" and the number of the wells 101 that is a cut line for determining whether or not a living body is infected with a virus is "B", B/A is preferably 0.2 or more, and more preferably 0.3 or more. By setting a cut line so that B/A is within the above range, it is possible to further improve the correctness of the result of determination (i.e., accuracy of determination) of the presence or absence of the anti-virus antibody in a living body.

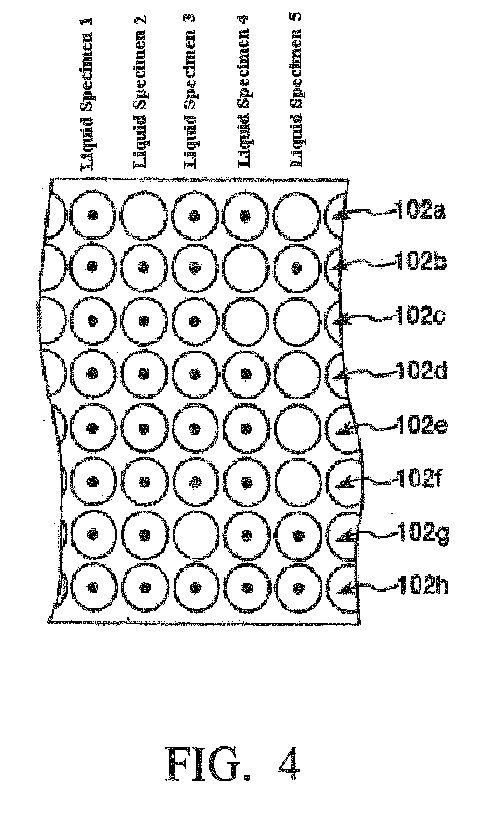

For example, on condition that when an antibody bound to the antigen 102 (102a to 102h) is detected in two or more of the wells 101 (101a to 101h), a liquid specimen shall be determined to be positive, and when an antibody bound to the antigen 102 (102a to 102h) is detected in any one of the wells 101 (101a to 101h) or an antibody bound to the antigen 102 is not detected, a liquid specimen shall be determined to be negative, liquid specimens 1 to 3 shown in Fig. 4 are determined to be negative and liquid specimens 4 and 5 shown in Fig. 4 are determined to be positive. That is, living bodies which provided the samples used for the liquid specimens 4 and 5 are determined to be infected with a specific virus.

It is to be noted that if a liquid specimen containing the above-described antibodies which can show cross reaction is subjected to a determination of the presence or absence of the anti-virus antibody using a conventional plate in which all the first to eighth antigens 102a to 102h are fixed on the inner surface of one well, the possibility that the liquid specimen is determined to be positive is almost 100 % in spite of the fact that the liquid specimen is negative.

In the case of determination of the presence or absence of the anti-virus antibody associated with human rights, such as HIV infection, it is preferred that the possibility of such a mistaken determination should be reduced as lower as possible. For this reason, the present invention is particularly suitable for use in determination of the presence or absence of HIV infection.

As has been descried above, according to the present invention, it is possible to easily and simply determine the presence or absence of the anti-virus antibody with higher accuracy.

Further, in the case of determination of the presence or absence of HIV infection, that is, in the case of detecting an anti-HIV antibody as an anti-virus antibody 103 by the use of an HIV antigen 102' as an antigen 102, the determination kit described above may further contain, for example, the following antagonist containing a protein 106 which can inhibit the binding of an inhibitory substance 105, which interferes with the precipitation of the colored particles 1, to the HIV antigen 102'.

Here, a sample obtained from a living body (especially, blood serum) contains many kinds of inhibitory substances 105 which bind to the HIV antigen 102' and the anti-IgG antibody 104' to interfere with the precipitation of the colored particles 1.

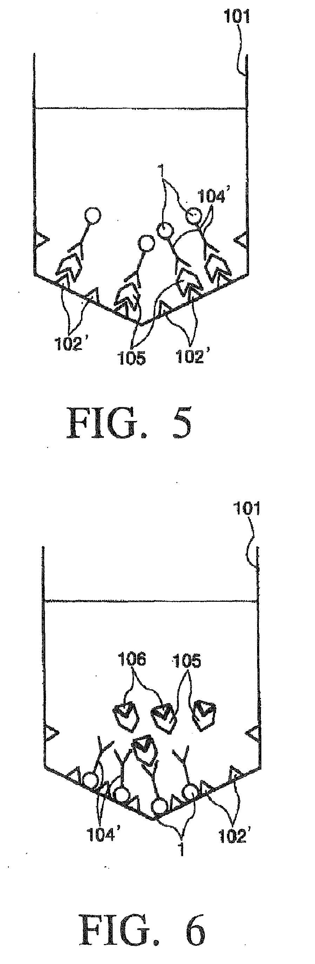

As shown in Fig. 5, in a case where such an inhibitory substance 105 has been bound to the HIV antigen 102' fixed on the inner surface of the well 101, the anti-IgG antibody 104' contained in the reagent fed into the well 101 is further bound to the inhibitory substance 105. As a result, the colored particles 1 are adsorbed to the inner surface of the well 101 through the anti-IgG antibodies 104', the inhibitory substances 105 and the HIV antigens 102' with the colored particles 1 being dispersed in the liquid in the well 101. This causes a mistaken determination that an anti-HIV antibody bound to the HIV antigen 102' is present in spite of the fact that such an anti-HIV antibody is not originally present.

In order to prevent such a mistaken determination, it is preferred that an antagonist containing a protein 106 having an affinity for the inhibitory substance 105 higher than that of the HIV antigen 102' is added to a liquid specimen before the liquid specimen is fed into the wells 101. As a result, the protein 106 is selectively (specifically) bound to the inhibitory substance 105 contained in the liquid specimen (i.e., in the sample). This prevents (inhibits) the binding of the inhibitory substance 105 contained in the liquid specimen fed into the well 101 to the HIV antigen 102' because, as shown in Fig. 6, the HIV antigen 102'-binding site of the inhibitory substance 105 is blocked by the protein 106.

Therefore, it is possible to prevent the occurrence of a phenomenon in which the anti-IgG antibody 104' is further bound to the inhibitory substance 105 bound to the HIV antigen 102' so that the colored particles 1 are dispersed in the liquid in the well 101 (which indicates that the liquid specimen is positive for HIV), that is, it is possible to reliably precipitate and agglutinate the colored particles 1, thereby reliably preventing a mistaken determination that an anti-HIV antibody is present in spite of the fact that it is not present.

The protein 106 is not particularly limited as long as it has an affinity for the inhibitory substance 105 higher than that of the HIV antigen 102' and has substantially no ability to bind to the HIV antigen 102', but is preferably at least one selected from the group comprising casein (phosphate protein), ovalbumin (glycoprotein), albumin, lactoferrin (glycoprotein) and chymotrypsinogen. Particularly, at least one selected from the group comprising casein, ovalbumin, and lactoferrin is preferably used. These proteins are preferred because their affinity for the inhibitory substance 105 is especially higher than that of the HIV antigen 102'.

The amount of the antagonist to be added to a liquid specimen is not particularly limited, but is preferably in the range of about 0.1 to 10 mg, and more preferably in the range of about 0.3 to 8 mg in terms of the amount of the protein 106, per $\mu$g of the HIV antigen 102'. By setting the amount of the protein 106 to be added to a liquid specimen to a value within the above range, it is possible to reliably reduce the amount of the inhibitory substance 105 not bound to the protein 106, thereby reliably interfering with the action of the inhibitory substance 105 that inhibits the precipitation of the colored particles 1 (hereinafter, simply referred to as "precipitation inhibitory action"). It is to be noted that even if the amount of the protein 106 to be added to a liquid specimen is increased to exceed the above upper limit value, it cannot be expected that the effect obtained by adding the protein 106 is further enhanced.

The protein 106 may be directly used as the antagonist, but it is preferred that the protein 106 is dissolved in an appropriate solvent before use. By doing so, the amount of the protein 106 to be added to a liquid specimen can be easily controlled.

Examples of the solvent include the buffers mentioned above with reference to the preparation of a reagent.

Preferably, the antagonist further contains a nonionic surfactant, which makes it possible to effectively interfere with the binding of the inhibitory substance 105 to the HIV antigen 102' or the anti-IgG antibody 104'.

Examples of the nonionic surfactant include Polyoxyethylene(9)-octylphenyl ether, polyoxyethylene(20)-sorbitan-monolaurate, polyoxyethylene(10)-octylphenyl ether, and n-nonanonyl-N-methylglucamide. These nonionic surfactants can be used singly or in combination of two or more of them.

By using such a determination kit, it is possible to inhibit the binding of the inhibitory substance 105 to the HIV antigen 102' by the action of the protein 106. As a result, in the case of a liquid specimen containing no anti-HIV antibody, the colored particles 1 are reliably sedimentated and agglutinated.

Further, since the precipitation inhibitory action of the inhibitory substance 105 is inhibited by the action of the protein 106, it is possible to eliminate the operation of cleaning for removing the inhibitory substance 105 from the wells 101, thereby improving operating efficiency and avoiding the risk that an operator becomes infected with HIV due to a liquid specimen or the like spattering around the operator during the operation of cleaning. Therefore, it is possible to ensure a high degree of security.

In a case where the antagonist is used, the dilution rate of a sample when a liquid specimen is prepared may be relatively low.

More specifically, in a case where blood serum is used as a sample, it is preferred that a liquid specimen is prepared by diluting blood serum about 2- to 1000-fold (particularly, 5- to 100-fold). Even when the dilution rate of the sample is such a relatively low value (that is, even when a relatively large amount of the inhibitory substance 105 is present in the liquid specimen), the use of the antagonist makes it possible to reliably precipitate and agglutinate the colored particles 1. Further, in the case of such a relatively low dilution rate, the operation of diluting a sample can be relatively easily carried out in a short period of time, thereby reducing the time required for determining the presence or absence of the anti-virus antibody.

Although the plate, the determination kit, and the determination method of the present invention have been described above, the present invention is not limited thereto.

For example, the determination method of the present invention may further contain any one or two or more steps, if necessary.

Further, in this embodiment, a case where an antibody is detected using particle agglutination has been described by way of example, but other detection methods may be alternatively used. For example, a liquid containing a labeled secondary antibody obtained by binding a label such as an enzyme, a radioactive material, or a fluorescent material to an anti-IgG antibody can be used as a reagent.

**Example**

Hereinbelow, an actual example of the present invention will be described.

(Example)

<1> First, a microplate containing 96 wells having a v-shaped bottom (capacity: 0.3 mL) was prepared. Then, each of the following 6 kinds of solutions of Ag1 to Ag6 (HIV antigens) was fed into different wells (50 μL/well) of the microplate to allow the inner surface of each well to carry one of the Ag1 to Ag6. It is to be noted that the wells carrying Ag1 to Ag6 on their respective inner surfaces in order were arranged in line in the microplate.

Ag1 recombinant HIV-1 IIIB gag p24 (manufactured by ImmunoDiagnostics): 1 μg/mL
Ag2 recombinant virus protein HIV-1 gp120 (manufactured by Advanced Biotechnologies): 0.5 μg/mL
Ag3 recombinant HIV-2 env(gp36) (manufactured by Virogen) : 1 μg/mL
Ag4 recombinant HIV-1 ensr(gp41-gp120) (manufactured by Virogen): 1μg/mL
Ag5 recombinant HIV-1 p17/24/gp120·gp41 (manufactured by Virogen): 1 μg/mL
Ag6 recombinant HIV-1/-2 env (manufactured by Virogen): 1 μg/mL

<2> Next, an antagonist was prepared by dissolving casein as protein and polyoxyethylene(20)-sorbitan-monolaurate ("Tween 20" manufactured by Wako Pure Chemical Industries) as a nonionic surfactant in a potassium dihydrogen phosphate-disodium hydrogen phosphate buffered saline solution so that the concentrations thereof were 3.4 mg/mL and 0.24 vol%, respectively.

<3> Then, negative liquid specimens were prepared by diluting previously prepared negative blood serums (samples) containing no anti-HIV antibody with the antagonist 100-fold. Positive liquid specimens were also prepared by diluting previously prepared positive blood serums (samples) containing an anti-HIV antibody with the antagonist 100-fold. It is to be noted that the negative blood serums used here had been determined to contain no anti-HIV antibody and the positive blood serums used here had been determined to contain an anti-HIV antibody by ELISA.

<4> Next, each of the negative and positive liquid specimens was fed into the wells in an amount of 0.05 mL per well. As a result, in the cases of Ag1 and Ag3 to Ag6 (HIV antigens), the amount of casein added per µg of the HIV antigen was 3.4 mg, and in the case of Ag2 (HIV antigen), the amount of casein added per µg of the HIV antigen was 6.8 mg.

It is to be noted that the amount of casein added per µg of Ag1 (HIV antigen) can be calculated as follows.

$$\text{Amount of casein per well} = 3.4 \text{ mg/mL} \times 0.05 \text{ mL} = 0.17 \text{ mg}$$

$$\text{Concentration of Ag1 (HIV antigen) solution fed into well} = 1 \text{ µg/mL}$$

$$\text{Amount of Ag1 (HIV antigen) per well} = 1 \text{ µg/mL} \times 50 \text{ µL} = 0.05 \text{ µg}$$

$$\text{Amount of casein per µg of Ag1 (HIV antigen) per well} = 0.17 \text{ mg} \div 0.05 \text{ µg} = 3.4 \text{ mg}$$

In the cases of Ag3 to Ag6, the amount of casein added per µg of the HIV antigen was the same as that in the case of Ag1. In the case of Ag2, the amount of casein added per µg of the HIV antigen was two times that in the case of Ag1. Then, a reagent containing nylon beads coated with hydroxyapatite (red colored particles) carrying an antibody (anti-IgG antibody) to be bound to an anti-HIV antibody was fed into the wells.

It is to be noted that the amount of the nylon beads coated with hydroxyapatite contained in the reagent was 0.1 wt%. The average particle diameter and the specific gravity of the nylon beads coated with hydroxyapatite were 5.8 µm and 1.13 g/cm$^3$, respectively.

<5> Next, the motion and state of the nylon beads coated with hydroxyapatite in each of the wells was observed.

In a case where precipitation and agglutination of the nylon beads coated with hydroxyapatite were not observed in two or more of the six wells, the liquid specimen was determined to be positive. In a case where precipitation and agglutination of the nylon beads coated with hydroxyapatite were not observed in one or less of the six wells, the liquid specimen was determined to be negative.

(Comparative Example)

The motion and state of the nylon beads coated with hydroxyapatite was observed in the same manner as in the Example except that the plate prepared in the step <1> was replaced with a plate prepared by feeding 50 µL of a mixture of solutions of Ag1 to Ag6 (HIV antigens) per well to allow the inner surface of each well to carry all the HIV antigens Ag1 to Ag6.

It is to be noted that the amount of casein added per µg of the HIV antigens (i.e., per µg of total amount of the HIV antigens Ag1 to Ag6) was calculated in the same manner as in the Example and was found to be 0.618 mg.

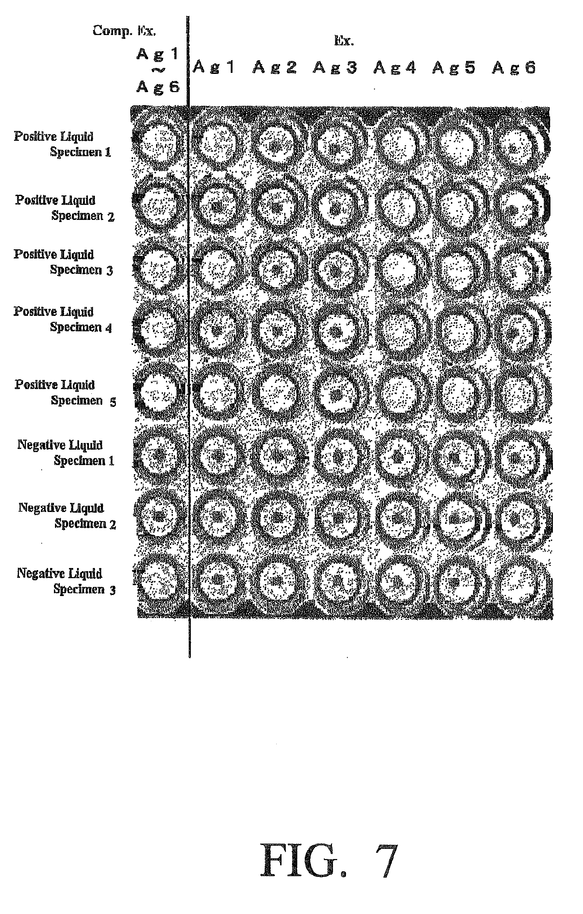

The results are shown in Fig. 7.

As shown in Fig. 7, in the Example, all the negative liquid specimens showed results that should be determined to be negative, and all the positive liquid specimens showed results that should be determined to be positive.

On the other hand, in the Comparative Example, one of the negative liquid specimens showed a result that should be determined to be positive, This is because the negative liquid specimen 3 contained an antibody which was not an anti-HIV antibody but could bind to Ag6.

**Effect of the Invention**

According to the present invention, it is possible to avoid a mistake that a sample without the anti-virus antibody (i.e., negative for virus) is determined to be positive. That is, it is possible to determine the presence or absence of the anti-virus antibody with higher accuracy.

Therefore, the present invention can be applied not only to screening for the anti-virus antibody but also to confirmed diagnosis.

Finally, it is also to be understood that the present disclosure relates to subject matter contained in Japanese Patent Application No. 2005-306420 (filed on October 20, 2005) which is expressly incorporated herein by reference in its entirety.

**Claims**

1. A plate to be used for determining whether or not a living body is infected with a specific virus, comprising:

   a plurality of wells into which a liquid specimen containing a sample obtained from the living body is to be fed, each of the wells having an inner surface; and
   two or more kinds of antigens derived from the virus, wherein each of the wells carries one of the two or more kinds of antigens on the inner surface thereof.

2. The plate as claimed in claim 1, wherein the plurality of wells include a plurality of wells arranged in line into which the same liquid specimen is to be fed.

3. The plate as claimed in claim 1, wherein the virus is HIV (Human Immunodeficiency Virus).

4. A determination kit to be used for determining whether or not a living body is infected with a specific virus, comprising:

   the plate defined by claims 1; and
   a reagent capable of detecting an antibody bound to the antigen.

5. The determination kit as claimed in claim 4, wherein the reagent contains particles carrying an anti-IgG antibody.

6. The determination kit as claimed in claim 5, wherein the particles are colored.

7. The determination kit as claimed in claim 5, wherein the bottom of each well in the plate is made convergent.

8. A determination method for determining whether or not a living body is infected with a specific virus using the determination kit defined by claims 4, the method comprising:

   feeding the liquid specimen into each of the predetermined wells in the plate;
   feeding the reagent into each of the wells containing the liquid specimen; and
   determining that the living body is infected with the virus when an antibody bound to the antigen is detected in a predetermined number or more of the wells.

9. The determination method as claimed in claim 8, wherein when the number of kinds of the antigens is defined as "A" and the predetermined number of the wells at which the living body is to be determined as being infected with the virus is defined as "B". B/A is 0.2 or higher.

FIG. 1

EP 1 777 522 A1

FIG. 2

FIG. 3

FIG. 4

FIG. 5

FIG. 6

FIG. 7

EP 1 777 522 A1

**European Patent Office**

**EUROPEAN SEARCH REPORT**

Application Number

EP 06 12 2690

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/45395 A (UNIVERSAL HEALTHWATCH INC [US]; CHOWDURY MOHAMMED A [US]; LOVCHIK JANE) 10 September 1999 (1999-09-10) * the whole document * in particular: * abstract * * page 1, paragraph 1 * * page 7, paragraph 1 * * page 21, paragraph 3 * * page 23, paragraph 1 - paragraph 2 * * page 26, paragraph 3; example 2 * ----- | 1-8 | INV. G01N33/569 B01L3/00 |
| X | WO 2004/092724 A2 (US GOVERNMENT [US]; KALISH MARCIA L [US]; NDONGMO CLEMENT B [US]; PAU) 28 October 2004 (2004-10-28) * the whole document * in particular: * abstract * * page 2, line 31 - line 33 * * page 12, line 12 - line 16 * * page 16, line 20 - line 31 * ----- | 1-8 | |
| A | US 5 994 149 A (ROBINSON HOWARD N [US] ET AL) 30 November 1999 (1999-11-30) * the whole document * in particular: * column 1, line 8 - line 9 * * column 3, line 45 - line 51 * * column 5, line 6 - line 50 * ----- -/-- | 1-8 | TECHNICAL FIELDS SEARCHED (IPC) G01N B01L |

The present search report has been drawn up for all claims

| Place of search | Date of completion of the search | Examiner |
|---|---|---|
| The Hague | 15 February 2007 | Gall, Anne-Laure |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................

& : member of the same patent family, corresponding document

EPO FORM 1503 03.82 (P04C01)

**European Patent Office**

**EUROPEAN SEARCH REPORT**

Application Number

EP 06 12 2690

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2004/101730 A (UNIV TSINGHUA [CN]; CAPITAL BIOCHIP COMPANY LTD [CN]; TAO SHENGCE [CN]) 25 November 2004 (2004-11-25) * the whole document * in particular: * abstract * * page 5, line 8 - line 22 * * page 26, line 8 - page 27, line 14; figure 2 * | 1-8 | |

TECHNICAL FIELDS SEARCHED (IPC)

The present search report has been drawn up for all claims

| Place of search | Date of completion of the search | Examiner |
|---|---|---|
| The Hague | 15 February 2007 | Gall, Anne-Laure |

## ANNEX TO THE EUROPEAN SEARCH REPORT
## ON EUROPEAN PATENT APPLICATION NO.

EP 06 12 2690

This annex lists the patent family members relating to the patent documents cited in the above-mentioned European search report.
The members are as contained in the European Patent Office EDP file on
The European Patent Office is in no way liable for these particulars which are merely given for the purpose of information.

15-02-2007

| Patent document cited in search report | | Publication date | Patent family member(s) | | Publication date |
|---|---|---|---|---|---|
| WO 9945395 | A | 10-09-1999 | AU 2790899 A<br>CA 2288869 A1<br>EP 0993615 A1 | | 20-09-1999<br>10-09-1999<br>19-04-2000 |
| WO 2004092724 | A2 | 28-10-2004 | AU 2004230606 A1<br>CA 2525640 A1<br>EP 1613964 A2 | | 28-10-2004<br>28-10-2004<br>11-01-2006 |
| US 5994149 | A | 30-11-1999 | US 6300140 B1 | | 09-10-2001 |
| WO 2004101730 | A | 25-11-2004 | AU 2003254595 A1<br>CN 1548549 A<br>EP 1627038 A1<br>JP 2006526134 T | | 03-12-2004<br>24-11-2004<br>22-02-2006<br>16-11-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5540995 A
- JP 2005306420 A